# EUROPEAN PATENT SPECIFICATION

(11) **EP 3 808 409 B1**
(45) Date of publication and mention of the grant of the patent: **06.05.2026**
(21) Application number: 19852145.2
(22) Date of filing: 05.08.2019
(51) Int. Cl.: A61N 5/10

(54) **RADIOTHERAPY APPARATUS**
STRAHLENTHERAPIEVORRICHTUNG
APPAREIL DE RADIOTHÉRAPIE

(30) Priority: 24.08.2018 CN 201810977447
(43) Date of publication of application: 21.04.2021
(73) Proprietor: Our United Corporation, Xi'an, Shaanxi 710018 (CN)
(72) Inventor: LIU, Haifeng, Xi'an, Shaanxi 710018 (CN); LI, Daliang, Xi'an, Shaanxi 710018 (CN)
(74) Representative: Kraus & Lederer PartGmbB
(86) International application number: PCT/CN2019/099312
(87) International publication number: WO 2020/038220

(56) References cited:
- EP-A1- 1 642 618
- EP-A1- 3 517 171
- EP-A1- 3 827 880
- WO-A1-2008/106468
- WO-A1-2018/056146
- CN-A- 101 541 236
- CN-A- 102 430 206
- CN-A- 102 430 206
- CN-A- 106 163 613
- CN-A- 108 635 687
- CN-U- 203 694 431
- CN-U- 203 694 431
- US-B1- 7 502 443

## Description

### TECHNICAL FIELD

The present disclosure relates to the field of medical equipment technologies, and in particular to a radiotherapy device.

### BACKGROUND

At present, in order to improve the speed and precision of tumor positioning to radiotherapy, a radiotherapy device usually simply combines a radiotherapy unit and an imaging unit, such that a patient is not required to be moved from an imaging chamber with the imaging unit to a treatment chamber with the radiotherapy unit, thereby realizing radiotherapy of a tumor of the patient.

For example, first, the patient is sent to the imaging unit for imaging by moving a position of the treatment couch in the radiotherapy device, then a treatment plan is formulated based on a size, a shape, surrounding tissues, and the like of the tumor in the imaging; and then, the patient is sent to the radiotherapy unit by moving the treatment couch and the treatment couch is positioned, such that the tumor position is consistent with the tumor position in the treatment plan, thereby realizing radiotherapy of the tumor of the patient. A system combining a radiotherapy unit and an imaging unit is described in publication CN 102 430 206 A.

### SUMMARY

The invention is defined by the claims. Embodiments of the present disclosure provide a radiotherapy device, including: a radiotherapy unit configured to emit a treatment beam to a to-be-treated area of a patient, the to-be-treated area of the patient being located outside the radiotherapy unit; and an imaging unit arranged adjacent to the radiotherapy unit and configured to emit an imaging beam to the to-be-treated area of the patient.

Alternatively, the radiotherapy unit includes a first radiotherapy unit and a second radiotherapy unit, and the imaging unit is located between the first radiotherapy unit and the second radiotherapy unit.

The radiotherapy unit includes: a radioactive source, a treatment beam emitted by the radioactive source is focused on an intersection point located outside the radiotherapy unit, and the intersection point coincides with an imaging center of the imaging unit.

Alternatively, the radiotherapy device further includes: a radioactive source receiving unit configured to receive and store the radioactive source when the radiotherapy device is not in operation.

The radiotherapy unit further includes: a shielding body, a source carrier, and a collimator which are covered together in sequence, the radioactive source is located on the source carrier; and the treatment beam emitted by the radioactive source is focused on the intersection point through the collimator.

The source carrier and/or the collimator are rotatable around a rotation axis, and/or movable along a preset trajectory.

The imaging unit is provided on an end surface on an edge of the source carrier or the collimator.

Alternatively, the radiotherapy unit is bowl-shaped or tube-shaped.

Alternatively, the radioactive source is uniformly distributed on the source carrier in a spiral shape or uniformly distributed on the source carrier in a peripheral direction.

Alternatively, the radiotherapy device further includes: a first anti-sinking unit provided between the shielding body and the source carrier.

Alternatively, the radiotherapy device further includes: a second anti-sinking unit provided between the source carrier and the collimator.

Alternatively, both the first anti-sinking unit and the second anti-sinking unit are bearings.

Alternatively, the radioactive source is arranged on a source box, and the source carrier includes: a source box mounting hole within which the source box is mounted.

Alternatively, the shielding body includes: a source box shielding hole and a source box shielding block, a size of the source box shielding hole is greater than or equal to a size of the radioactive source mounting hole, and the source box shielding block is adapted to the source box shielding hole.

Alternatively, the imaging unit includes an imaging source and an imager, a center axis of an imaging beam emitted by the imaging source deviates from a reference axis, and the reference axis is an axis that passes through the imaging center and is perpendicular to the imager.

Alternatively, the imaging unit includes a shielding member arranged adjacent to the radiotherapy unit and configured to shield the treatment beam passing through the intersection point.

Alternatively, the shielding member is hollow-shaped or sheet-shaped.

Alternatively, the imaging unit is at least one of: an X-ray apparatus, a CT apparatus, an MRI apparatus, a PET apparatus, an ultrasonic apparatus, or a DSA apparatus.

Alternatively, the radiotherapy device further includes: a shielding door configured to turn on or off the radiotherapy device, and/or shield the treatment beam emitted from the radiotherapy unit.

Alternatively, the radiotherapy device further includes: a treatment couch arranged at a side of the imaging unit.

### BRIEF DESCRIPTION OF THE DRAWINGS

The accompanying drawings are used for providing further understanding of technical solutions of the present disclosure, constitute a part of the DESCRIPTION, and are used for interpreting the technical solutions of the present disclosure together with embodiments of the present disclosure, but do not impose any limitation on the technical solutions of the present disclosure.
FIG. 1 is a schematic side view of a radiotherapy device provided in an embodiment of the present disclosure;
FIG. 2 is a schematic side view of another radiotherapy device provided in an embodiment of the present disclosure;
FIG. 3 is a schematic side view of still another radiotherapy device provided in an embodiment of the present disclosure;
FIG. 4 is a schematic side view of yet another radiotherapy device provided in an embodiment of the present disclosure;
FIG. 5 is a schematic side view of a radiotherapy device provided in another embodiment of the present disclosure;
FIG. 6 is a schematic diagram of a relationship between a center axis of an imaging beam and a reference axis provided by an embodiment of the disclosure;
FIG. 7 is a schematic side view of another radiotherapy device provided in another embodiment of the present disclosure;
FIG. 8 is a schematic side view of still another radiotherapy device provided in still another embodiment of the present disclosure; and
FIG. 9 is a schematic side view of yet another radiotherapy device provided in yet another embodiment of the present disclosure.

### DETAILED DESCRIPTION

In order to make the objectives, technical solutions, and advantages of the present disclosure clearer, embodiments of the present disclosure will be described in detail below with reference to the accompanying drawings. It should be noted that some embodiments in the present disclosure and some features in the embodiments of the present disclosure may be combined with each other in any manner on a non-conflict basis.

In the related art, before a radiotherapy device performs radiotherapy on a tumor of a patient, a treatment couch needs to be repositioned because an imaging unit of the treatment couch is moved to a radiotherapy unit. This will result in a positioning error of the treatment couch, and result in low precision of radiotherapy. In addition, the imaging unit and a radiotherapy apparatus in the radiotherapy device cannot work simultaneously, thereby failing in real-time image guidance on a patient, especially a patient in a process of radiotherapy.

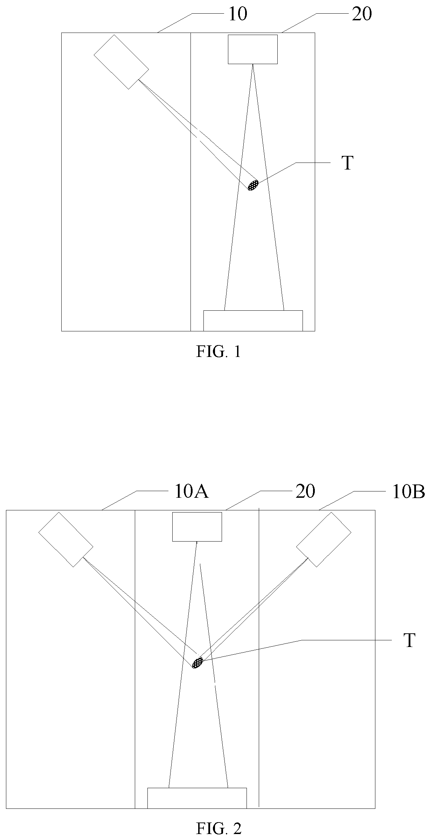

FIG. 1 is a schematic side view of a radiotherapy device provided in an embodiment of the present disclosure. As shown in FIG. 1, the radiotherapy device includes: a radiotherapy unit 10 and an imaging unit 20.

The radiotherapy unit 10 is configured to emit a treatment beam to a to-be-treated area T of a patient, where the to-be-treated area T of the patient is located outside the radiotherapy unit 10. The imaging unit 20 is arranged adjacent to the radiotherapy unit 10, and is configured to emit an imaging beam to the to-be-treated area T of the patient.

The radiotherapy device in the embodiment of the present disclosure sends the imaging beam to the to-be-treated area T of the patient using the imaging unit 20, to acquire imaging of the to-be-treated area T of the patient, and formulate a treatment plan using the imaging of the to-be-treated area T of the patient. The radiotherapy unit 10 may also emit the treatment beam to the same to-be-treated area T, i.e., a projection object of the radiotherapy unit 10 is the same as that of the imaging unit 20, which are both the to-be-treated area T. Therefore, without moving a position of the to-be-treated area of the patient, the radiotherapy unit 10 can emit the treatment beam to the to-be-treated area T of the patient for radiotherapy based on the formulated treatment plan, thereby efficiently improving the precision of radiotherapy. In addition, during radiotherapy, the imaging unit 20 and the radiotherapy unit 10 in the radiotherapy device can work simultaneously (i.e., treating whilst imaging) to realize real-time image guidance on the to-be-treated area T of the patient, thereby ensuring that the position of the to-be-treated area T is always consistent with that in the treatment plan.

Further, there may be a plurality of radiotherapy units 10. For example, as shown in FIG. 2, the radiotherapy unit 10 may include a first radiotherapy unit 10A and a second radiotherapy unit 10B. The imaging unit 20 is located between the first radiotherapy unit 10A and the second radiotherapy unit 10B. The first radiotherapy unit 10A and the second radiotherapy unit 10B can emit the treatment beam to the to-be-treated area T simultaneously, so as to improve the efficiency of radiotherapy.

In the embodiment of the present disclosure, the radiotherapy unit 10 may be, e.g., a conformal intensity-modulated radiotherapy apparatus, a cyberknife (X-knife), or a multi-source focused radiotherapy apparatus. The imaging unit 20 may be at least one of: an X-ray apparatus, a Cone Beam CT (CBCT) apparatus, a Computed Tomography (CT) apparatus, a Magnetic Resonance Imaging (MRI) apparatus, a Positron Emission Computed Tomography (PET) apparatus, an ultrasonic apparatus, or a Digital Subtraction Angiography (DAS) apparatus.

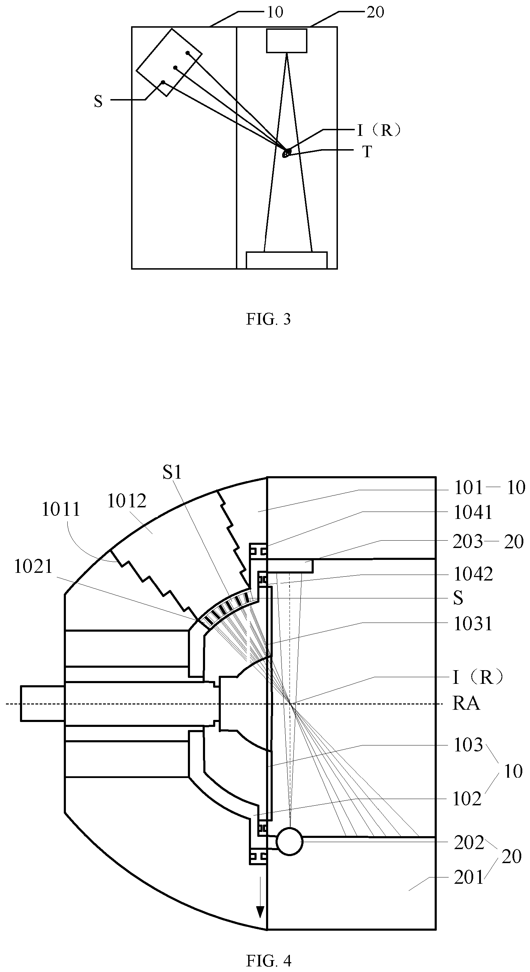

When the radiotherapy unit 10 is the multi-source focused radiotherapy apparatus, as shown in FIG. 3, the radiotherapy unit 10 includes a plurality of radioactive sources S. The treatment beam emitted by the plurality of radioactive sources S are focused on an intersection point I located outside the radiotherapy unit 10, and the intersection point I coincides with an imaging center R of the imaging unit 20. Accordingly, when there are two radiotherapy units 10, the first radiotherapy unit 10A and the second radiotherapy unit 10B each include a plurality of radioactive sources S, the treatment beams emitted by the plurality of radioactive sources S in the first radiotherapy unit 10A and the second radiation unit 10B are all focused on the intersection point I, and the intersection point I coincides with the imaging center R of the imaging unit 20.

It should be noted that the to-be-treated area T of the patient may include one or more to-be-treated target sites. For each radiotherapy, the intersection point I where a plurality of treatment beams is focused coincides with one of the target sites thereof.

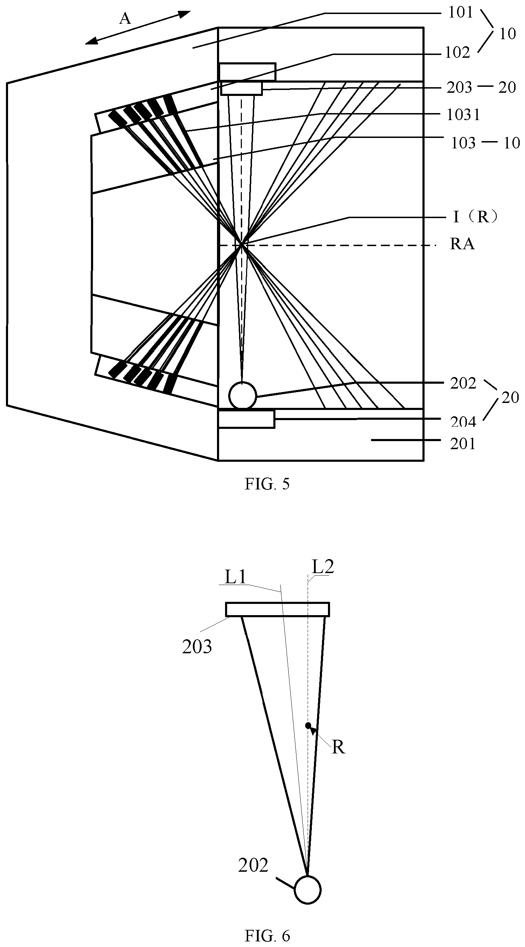

Further, as shown in FIG. 4 and FIG. 5, the radiotherapy unit 10 includes: a shielding body 101, a source carrier 102, and a collimator 103 in sequence from outside to inside. The shielding body 101 is configured to shield rays, the source carrier 102 is configured to bear a plurality of radioactive sources S, and the collimator 103 has a plurality of collimating channels 1031. There is one-to-one correspondence between the plurality of radioactive sources S and the plurality of collimating channels 1031. The radiotherapy unit 10 has an open source state and a closed source state.

When the radiotherapy unit 10 is in the open source state, each collimating channel 1031 of the collimator 103 is aligned with a corresponding radiation source S, i.e., the plurality of radioactive sources S is turned on, and the treatment beam emitted by the plurality of radioactive sources S may be focused on the intersection point I through the plurality of collimating channels 1031 to treat the target sites of the patient.

When the radiotherapy unit 10 is in the closed source state, each collimating channel 1031 of the collimator 103 is misaligned with the corresponding radioactive source S, i.e., the plurality of radioactive sources S is turned off, the treatment beam emitted by the plurality of radioactive source S is shielded by the collimator 103, and the radiotherapy device stops treatment.

In order to realize switching between the open source state and the closed source state of the radiotherapy unit 10, the embodiment of the present disclosure may be implemented through at least three possible examples as follows:

In a first possible example, as shown in FIG. 4, the radiotherapy unit 10 is configured to realize switching between the open source state and the closed source state by the source carrier 102 and/or the collimator 103 rotating around a rotation axis RA.

For example, the source carrier 102 in the radiotherapy unit 10 does not rotate around the rotation axis RA, while the collimator 103 rotates around the rotation axis RA, and the rotation of the collimator 103 makes each collimating channel 1031 of the collimator 103 be aligned or misaligned with the corresponding radioactive source S, thereby realizing switching between the open source state and the closed source state. Similarly, the source carrier 102 may rotate around the rotation axis RA, while the collimator 103 does not rotate around the rotation axis RA, thereby realizing switching between the open source state and the closed source state. Of course, the source carrier 102 and the collimator 103 rotate around the rotation axis RA in different rotation directions or at different rotation speeds, which can also realize switching between the open source state and the closed source state.

In the case where each collimating channel 1031 of the collimator 103 is aligned with the corresponding radioactive source S, if the source carrier 102 and the collimator 103 rotate around the rotation axis RA together, rays (i.e., the treatment beam) emitted by the radioactive source S irradiate the target site at different angles, thereby preventing normal tissues around the target sites from prolonged exposure to ray radiation.

In a second possible example, as shown in FIG. 5, the radiotherapy unit 10 is configured to realize switching between the open source state and the closed source state by the source carrier 102 and/or the collimator 103 moving along a preset trajectory A.

For example, the source carrier 102 in the radiotherapy unit 10 does not move along the preset trajectory A, while the collimator 103 moves along the preset trajectory A, and the movement of the collimator 103 makes each collimating channel 1031 of the collimator 103 be aligned or misaligned with the corresponding radioactive source S. Similarly, the source carrier 102 may move along the preset trajectory A, while the collimator 103 does not move along the preset trajectory A, thereby realizing switching between the open source state and the closed source state. Of course, the source carrier 102 and the collimator 103 move along the preset trajectory A in different moving directions or at different movement speeds, which can also realize switching between the open source state and the closed source state.

In a third possible example, as shown in FIG. 5, the radiotherapy unit 10 is configured to realize switching between the open source state and the closed source state by the source carrier 102 and/or the collimator 103 rotating around the rotation axis RA, and by the source carrier 102 and/ or the collimator 103 moving along the preset trajectory A. It should be noted that, the first possible example and the second possible example may be referred to for an implementation of the third possible example. The description will not be repeated in the embodiment of the present disclosure.

It should be noted that the first example takes a bowl-shaped radiotherapy unit 10 as an example, and the second example and the third example take a tube-shaped radiotherapy unit 10 as an example. Of course, the radiotherapy unit 10 may also have other structures, and its shape is not specifically limited in the embodiment of the present disclosure.

Alternatively, the shielding body 101 of the radiotherapy unit 10 is usually made of a shielding material such as lead, or tungsten. A total weight of the radiotherapy unit 10 is very large. Due to the effect of gravity, as shown in FIG. 4, the radiotherapy unit 10 will sink in a direction perpendicular to the rotation axis RA (an arrow direction in the figure). Therefore, a first anti-sinking component 1041 may be provided between the shielding body 101 and the source carrier 102, and a second anti-sinking component 1042 may be provided between the source carrier 102 and the collimator 103. The first anti-sinking component 1041 and the second anti-sinking component 1042 can prevent the radiotherapy unit 10 from integrally sinking. Further, the first anti-sinking component 1041 and the second anti-sinking component 1042 may both be annular bearings, e.g., and may be rolling bearings.

In the embodiment of the present disclosure, in order to facilitate the installation or replacement of the radioactive source, the bowl-shaped radiotherapy unit 10 is taken as an example. As shown in FIG. 4, the radioactive source S may be arranged on a source box S1. Accordingly, the source carrier 102 includes a source box mounting hole 1021, and the source box S1 is mounted within the source box mounting hole 1021. Further, the shielding body 101 includes: a source box shielding hole 1011 and a source box shielding block 1012, where a size of the source box shielding hole 1011 is greater than or equal to a size of the source box mounting hole 1021, the source box shielding block 1012 is adapted to the source box shielding hole 1011, and the source box shielding block 1012 is configured to block the source box shielding hole 1011 to realize shielding the treatment beam emitted by the radioactive source S.

Alternatively, the radioactive sources S mentioned above may be uniformly distributed on the source carrier 102 in a spiral shape. The radioactive sources S may also be divided into a plurality of groups, and may all be distributed on a fan-shaped surface of the source carrier 102. Each group of radioactive sources is arranged along a direction of the rotation axis RA, or each group of radioactive sources is uniformly distributed along a peripheral direction of a ring-shaped shielding member 201 of the source carrier 102.

It should be noted that no matter how the radioactive source is turned on or off, the treatment beam passing through the intersection point I may leak out. Therefore, as shown in FIG. 4 and FIG. 5, the imaging unit 20 includes the shielding member 201. The shielding member 201 is arranged adjacent to the radiotherapy unit 20. The shielding member 201 is configured to shield the treatment beam passing through the intersection point I. The treatment beam emitted by the radioactive source S can be blocked by the shielding member 201 after passing through the intersection point I, thereby effectively preventing the rays from leaking during treatment.

The shielding member 201 mentioned above may be hollow-shaped, e.g., ring-shaped; or the shielding member 201 may be sheet-shaped, e.g., C-shaped. A sheet-shaped shielding member 201 can rotate with the radioactive source S to shield the rays (the treatment beam) emitted by the radioactive source S at any time, but a thickness of the shielding member 201, a size of a middle opening of a hollow-shaped shielding member, and a size of the sheet-shaped shielding member may be set based on a direction and an intensity of the treatment beam passing through the intersection point I.

Further, the imaging unit 20 further includes: an imaging source 202 and an imager 203. In a possible example, as shown in FIG. 4, the imaging source 202 and the imager 203 are arranged oppositely on an end surface on an edge of the source carrier 102 or the collimator 103, and the imaging source 202 and the imager 203 can rotate with the rotation of the source carrier 102 or the collimator 103 around the rotation axis Ra. In another possible example, as shown in FIG. 5, the imaging source 202 and the imager 203 in the imaging unit 20 are arranged oppositely on a rotating ring 204 within the shielding member 201, and the imaging source 202 and the imager 203 can rotate around the rotation axis RA. In other possible examples, there are at least two groups of imaging sources 202 and imagers 203 in the imaging unit 20. When there are two groups of imaging sources 202 and imagers 203 in the imaging unit 20, the two groups of imaging sources 202 and imagers 203 are arranged oppositely within the shielding member 201 respectively, and there is a preset included angle between the two groups of imaging sources 202 and imagers 203. For example, the preset included angle is 90 degrees.

Further, in order to increase an imaging volume, as shown in FIG. 6, a center axis L1 of an imaging beam emitted by the imaging source 202 deviates from a reference axis L2. The reference axis L2 is an axis that passes through the imaging center R and is perpendicular to the imager 203. When the imaging unit 20 (e.g., the imaging source 202 and the imager 203) rotates with the radiotherapy unit 10, the imaging beam will form a larger imaging volume.

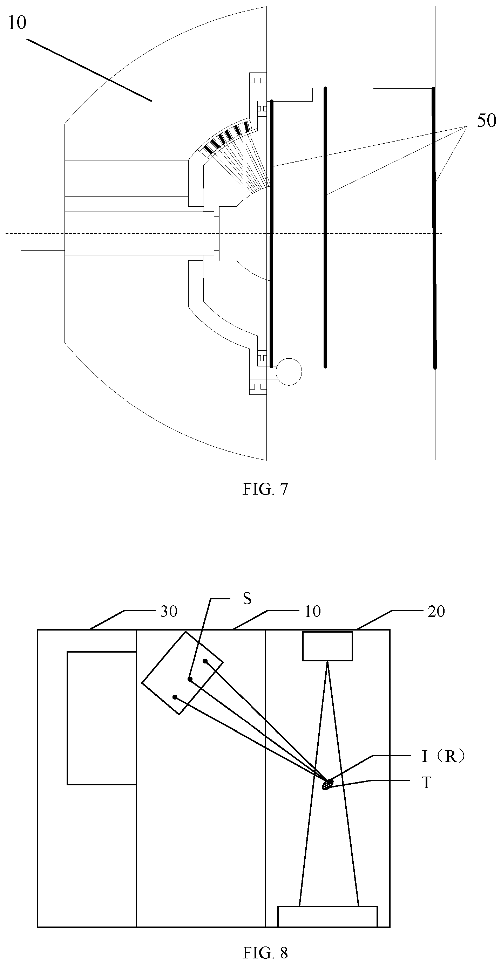

Further, as shown in FIG. 7, the radiotherapy device further includes: a shielding door 50. The shielding door 50 is configured to turn on or off the radiotherapy device, and/or shield the treatment beam emitted from the radiotherapy unit 10. It should be noted that FIG. 7 is schematic description by taking the bowl-shaped radiotherapy unit 10 shown in FIG. 3 as an example, and the shielding door 50 may be arranged at an exit of the treatment beam of the radiotherapy unit 10 and/or inside the imaging unit 20 and/or outside the imaging unit 20. When the radiotherapy device is not in operation, in the case where the beam emitted by the radioactive source S is not completely shielded by the collimator 103, the shielding door 50 can be used to shield these unshielded rays; and in the case where the beam emitted by the radioactive source S is completely shielded by the collimator 103, the shielding door 50 can be used to open or close a treatment space of the radiotherapy device. Of course, the shielding door 50 can also be used to open or close the treatment space of the radiotherapy device whilst shielding these unshielded rays.

In the embodiment of the present disclosure, when the radiotherapy unit 10 in the above embodiments is of a semi-shielding design, in order to avoid leakage of the radioactive source S when the radiotherapy unit 10 is not used, as shown in FIG. 8, the radiotherapy device further includes: a radioactive source receiving unit 30 configured to receive and store the radioactive source S within the radioactive source receiving unit 30 when the radiotherapy device 10 is not in operation.

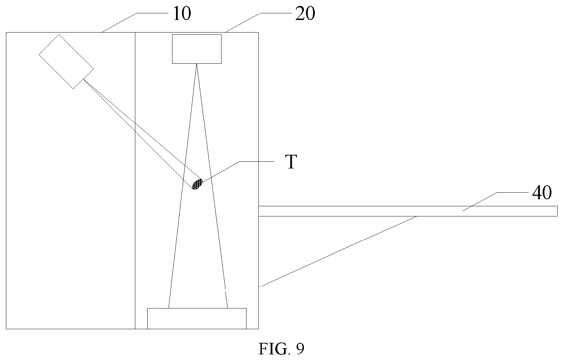

Alternatively, as shown in FIG. 9, the radiotherapy device may further include: a treatment couch 40. The treatment couch 40 is arranged at a side of the imaging unit 20, and the treatment couch 40 may be a three-dimensional couch or a six-dimensional couch.

In conclusion, the radiotherapy device in the embodiments of the present disclosure sends an imaging beam to a to-be-treated area of a patient using an imaging unit, to acquire imaging of the to-be-treated area of the patient, and formulates a treatment plan using the imaging of the to-be-treated area of the patient. A radiotherapy unit may also emit a treatment beam to the same to-be-treated area, i.e., a projection object of the radiotherapy unit is the same as that of the imaging unit, which are both the to-be-treated area. Therefore, without moving a position of the to-be-treated area of the patient, the radiotherapy unit can emit the treatment beam to the to-be-treated area of the patient for radiotherapy based on the formulated treatment plan, thereby efficiently improving the precision of radiotherapy. In addition, during radiotherapy, the imaging unit and the radiotherapy unit in the radiotherapy device can work simultaneously (i.e., treating whilst imaging) to realize real-time image guidance on the to-be-treated area of the patient, thereby ensuring that the position of the to-be-treated area is always consistent with that in the treatment plan.

The above description is only provided to facilitate understanding the technical solutions of the present disclosure by those skilled in the art, and is not intended to limit the present disclosure. The invention is defined by the appended claims.

## Claims

1. A radiotherapy device, comprising:
a radiotherapy unit (10) configured to emit a treatment beam to a to-be-treated area (T) of a patient, wherein in use the to-be-treated area (T) of the patient is located outside the radiotherapy unit (10); and wherein the to-be treated area (T) of the patient includes one or more to-be-treated target sites; and
an imaging unit (20) arranged adjacent to the radiotherapy unit (10) and configured to emit an imaging beam to the to-be-treated area (T) of the patient,
wherein the radiotherapy unit (10) comprises: a radioactive source (S) configured to emit a treatment beam;
wherein the radiotherapy unit further comprises:
a shielding body (101), a source carrier (102), and a collimator (103) arranged in sequence from outside to inside, wherein the shielding body (101) covers the source carrier (102), the source carrier (102) covers the collimator (103), the radioactive source (S) is located on the source carrier (102); and the treatment beam emitted by the radioactive source (S) is focused on an intersection point (I) located outside the radiotherapy unit (10) through the collimator (103), and the intersection point coincides with an imaging center of the imaging unit;
wherein the source carrier (102) and/or the collimator (103) are rotatable around a rotation axis(RA), wherein in use the intersection point (I) coincides with one of the target sites of the to-be-treated area (T) of the patient, and
the imaging unit (20) is arranged on an end surface on an edge of the source carrier (102) or the collimator (103) so that the imaging unit (20) can rotate with the rotation of the source carrier (102) or the collimator (103) around the rotation axis (Ra).

2. The radiotherapy device according to claim 1, wherein the radiotherapy unit (10) comprises:
a first radiotherapy unit (10A) and a second radiotherapy unit (10B), and the imaging unit (20) is located between the first radiotherapy unit (10A) and the second radiotherapy unit.

3. The radiotherapy device according to claim 1, further comprising: a radioactive source receiving unit (30) configured to receive and store the radioactive source (S) when the radiotherapy device is not in operation.

4. The radiotherapy device according to claim 1, further comprising: a first anti-sinking unit (1041) provided between the shielding body (101) and the source carrier (102).

5. The radiotherapy device according to claim 4, further comprising: a second anti-sinking unit (1042) provided between the source carrier (101) and the collimator (103).

6. The radiotherapy device according to claim 1, wherein the radioactive source (S) is arranged on a source box (S1), and the source carrier (102) comprises: a source box mounting hole within which the source box (S1) is mounted.

7. The radiotherapy device according to claim 6, wherein the shielding body (101) comprises: a source box shielding hole (1011) and a source box shielding block (1012), a size of the source box shielding hole (1011) is greater than or equal to a size of the source box mounting hole, and the source box shielding block (1012) is adapted to the source box shielding hole (1011).

8. The radiotherapy device according to claim 1, wherein the imaging unit (20) comprises an imaging source (202) and an imager (203), a center axis of an imaging beam emitted by the imaging source (202) deviates from a reference axis, and the reference axis is an axis that passes through the imaging center (R) and is perpendicular to the imager (203).

9. The radiotherapy device according to claim 1, wherein the imaging unit (20) comprises a shielding member (201) arranged adjacent to the radiotherapy unit (10) and configured to shield the treatment beam passing through the intersection point (I).

10. The radiotherapy device according to claim 9, wherein the shielding member (201) is hollow-shaped or sheet-shaped.

11. The radiotherapy device according to claim 1, wherein the imaging unit (20) comprises at least one of:
an X-ray apparatus, a CT apparatus, an MRI apparatus, a PET apparatus, an ultrasonic apparatus, or a DSA apparatus.

12. The radiotherapy device according to claim 1, further comprising: a shielding door (50) configured to turn on or off the radiotherapy device, and/or shield the treatment beam emitted from the radiotherapy unit (10).

13. The radiotherapy device according to claim 1, wherein the radiotherapy unit (10) is bowl-shaped or tube-shaped.

## Patentansprüche

1. Strahlentherapievorrichtung, umfassend:
eine Strahlentherapieeinheit (10), die dazu konfiguriert ist, einen Behandlungsstrahl auf einen zu behandelnden Bereich (T) eines Patienten zu emittieren, wobei sich der zu behandelnde Bereich (T) des Patienten im Gebrauch außerhalb der Strahlentherapieeinheit (10) befindet; und wobei der zu behandelnde Bereich (T) des Patienten eine oder mehrere zu behandelnde Zielstellen umfasst; und
eine Bildgebungseinheit (20), die angrenzend an die Strahlentherapieeinheit (10) angeordnet ist und dazu konfiguriert ist, einen Bildgebungsstrahl auf den zu behandelnden Bereich (T) des Patienten zu emittieren,
wobei die Strahlentherapieeinheit (10) eine radioaktive Quelle (S) umfasst, die dazu konfiguriert ist, einen Behandlungsstrahl zu emittieren; wobei die Strahlentherapieeinheit ferner umfasst: einen Abschirmkörper (101), einen Quellenträger (102) und einen Kollimator (103), die der Reihe nach von außen nach innen angeordnet sind, wobei der Abschirmkörper (101) den Quellenträger (102) abdeckt, der Quellenträger (102) den Kollimator (103) abdeckt und sich die radioaktive Quelle (S) auf dem Quellenträger (102) befindet; und wobei der durch die radioaktive Quelle (S) emittierte Behandlungsstrahl durch den Kollimator (103) auf einen Schnittpunkt (I) fokussiert wird, der sich außerhalb der Strahlentherapieeinheit (10) befindet, und wobei der Schnittpunkt mit einem Bildgebungszentrum der Bildgebungseinheit zusammenfällt; wobei der Quellenträger (102) und/oder der Kollimator (103) um eine Drehachse (RA) drehbar sind, wobei im Gebrauch der Schnittpunkt (I) mit einer der Zielstellen des zu behandelnden Bereichs (T) des Patienten zusammenfällt, und
wobei die Bildgebungseinheit (20) auf einer Endfläche an einem Rand des Quellenträgers (102) oder des Kollimators (103) angeordnet ist, so dass sich die Bildgebungseinheit (20) mit der Drehung des Quellenträgers (102) oder des Kollimators (103) um die Drehachse (Ra) drehen kann.

2. Strahlentherapievorrichtung nach Anspruch 1, wobei die Strahlentherapieeinheit (10) umfasst:
eine erste Strahlentherapieeinheit (10A) und eine zweite Strahlentherapieeinheit (10B), wobei sich die Bildgebungseinheit (20) zwischen der ersten Strahlentherapieeinheit (10A) und der zweiten Strahlentherapieeinheit befindet.

3. Strahlentherapievorrichtung nach Anspruch 1, ferner umfassend: eine Einheit zur Aufnahme von radioaktiven Quellen (30), die so konfiguriert ist, dass sie im Fall, in dem die Strahlentherapievorrichtung nicht in Betrieb ist, die radioaktive Quelle (S) aufnimmt und lagert.

4. Strahlentherapievorrichtung nach Anspruch 1, ferner umfassend: eine erste Absenksicherungseinheit (1041), die zwischen dem Abschirmkörper (101) und dem Quellenträger (102) vorgesehen ist.

5. Strahlentherapievorrichtung nach Anspruch 4, ferner umfassend: eine zweite Absenksicherungseinheit (1042), die zwischen dem Quellenträger (101) und dem Kollimator (103) vorgesehen ist.

6. Strahlentherapievorrichtung nach Anspruch 1, wobei die radioaktive Quelle (S) an einem Quellenkasten (S1) angeordnet ist, und wobei der Quellenträger (102) ein Quellenkasten-Montageloch umfasst, in dem der Quellenkasten (S1) montiert ist.

7. Strahlentherapievorrichtung nach Anspruch 6, wobei der Abschirmkörper (101) ein Quellenkasten-Abschirmloch (1011) und einen Quellenkasten-Abschirmblock (1012) umfasst, wobei eine Größe des Quellenkasten-Abschirmlochs (1011) größer als oder gleich wie eine Größe des Quellenkasten-Montagelochs ist, und wobei der Quellenkasten-Abschirmblock (1012) an das Quellenkasten-Abschirmloch (1011) angepasst ist.

8. Strahlentherapievorrichtung nach Anspruch 1, wobei die Bildgebungseinheit (20) eine Bildgebungsquelle (202) und einen Bildgeber (203) umfasst, wobei eine Mittelachse eines von der Bildgebungsquelle (202) emittierten Bildgebungsstrahls von einer Referenzachse abweicht, und wobei die Referenzachse eine Achse ist, die durch das Bildgebungszentrum (R) verläuft und senkrecht zu dem Bildgeber (203) steht.

9. Strahlentherapievorrichtung nach Anspruch 1, wobei die Bildgebungseinheit (20) ein Abschirmelement (201) umfasst, das angrenzend an die Strahlentherapieeinheit (10) angeordnet ist und dazu konfiguriert ist, den durch den Schnittpunkt (I) verlaufenden Behandlungsstrahl abzuschirmen.

10. Strahlentherapievorrichtung nach Anspruch 9, wobei das Abschirmelement (201) hohl oder plattenförmig ist.

11. Strahlentherapievorrichtung nach Anspruch 1, wobei die Bildgebungseinheit (20) mindestens eines der folgenden Geräte umfasst:
ein Röntgengerät, ein CT-Gerät, ein MRT-Gerät, ein PET-Gerät, ein Ultraschallgerät oder ein DSA-Gerät.

12. Strahlentherapievorrichtung nach Anspruch 1, ferner umfassend: eine Abschirmtür (50), die dazu konfiguriert ist, die Strahlentherapievorrichtung ein- oder auszuschalten und/oder den von der Strahlentherapieeinheit (10) emittierten Behandlungsstrahl abzuschirmen.

13. Strahlentherapievorrichtung nach Anspruch 1, wobei die Strahlentherapieeinheit (10) schalenförmig oder rohrförmig ist.

## Revendications

1. Dispositif de radiothérapie, comprenant :
une unité de radiothérapie (10) configurée pour émettre un faisceau de traitement vers une zone à traiter (T) d'un patient, dans laquelle, en utilisation, la zone à traiter (T) du patient est située à l'extérieur de l'unité de radiothérapie (10) ; et dans laquelle la zone à traiter (T) du patient comprend un ou plusieurs sites cibles à traiter ; et
une unité d'imagerie (20) disposée pour être adjacente à l'unité de radiothérapie (10) et configurée pour émettre un faisceau d'imagerie vers la zone à traiter (T) du patient,
dans lequel l'unité de radiothérapie (10) comprend : une source radioactive (S) configurée pour émettre un faisceau de traitement ;
dans lequel l'unité de radiothérapie comprend en outre :
un corps de blindage (101), un porteur de source (102) et un collimateur (103) disposés en séquence de l'extérieur vers l'intérieur, dans lequel le corps de blindage (101) recouvre le porteur de source (102), le porteur de source (102) recouvre le collimateur (103), la source radioactive (S) est située sur le porteur de source (102) ; et le faisceau de traitement émis par la source radioactive (S) est focalisé à un point d'intersection (I) situé à l'extérieur de l'unité de radiothérapie (10) à travers le collimateur (103), et le point d'intersection coïncide avec un centre d'imagerie de l'unité d'imagerie ;
le porteur de source (102) et/ou le collimateur (103) sont rotatifs autour d'un axe de rotation (RA), dans lequel, en utilisation, le point d'intersection (I) coïncide avec l'un des sites cibles de la zone à traiter (T) du patient, et
l'unité d'imagerie (20) est disposée sur une surface terminale sur un bord du porteur de source (102) ou du collimateur (103) afin de permettre à l'unité d'imagerie (20) de tourner avec la rotation du porteur de source (102) ou du collimateur (103) autour de l'axe de rotation (RA).

2. Dispositif de radiothérapie selon la revendication 1, dans lequel l'unité de radiothérapie (10) comprend :
une première unité de radiothérapie (10A) et une seconde unité de radiothérapie (10B), et l'unité d'imagerie (20) est située entre la première unité de radiothérapie (10A) et la seconde unité de radiothérapie.

3. Dispositif de radiothérapie selon la revendication 1, comprenant en outre : une unité de réception de source radioactive (30) configurée pour recevoir et stocker la source radioactive (S) lorsque le dispositif de radiothérapie n'est pas en fonctionnement.

4. Dispositif de radiothérapie selon la revendication 1, comprenant en outre : une première unité anti-affaissement (1041) prévue entre le corps de blindage (101) et le porteur de source (102).

5. Dispositif de radiothérapie selon la revendication 4, comprenant en outre : une seconde unité anti-affaissement (1042) prévue entre le porteur de source (101) et le collimateur (103).

6. Dispositif de radiothérapie selon la revendication 1, dans lequel la source radioactive (S) est disposée sur une boîte à source (51), et le porteur de source (102) comprend : un trou de montage de boîte à source dans lequel est montée la boîte à source (51).

7. Dispositif de radiothérapie selon la revendication 6, dans lequel le corps de blindage (101) comprend : un trou de blindage de boîte à source (1011) et un bloc de blindage de boîte à source (1012), une taille du trou de blindage de boîte à source (1011) est supérieure ou égale à une taille du trou de montage de boîte à source, et le bloc de blindage de boîte à source (1012) est adapté au trou de blindage de boîte à source (1011).

8. Dispositif de radiothérapie selon la revendication 1, dans lequel l'unité d'imagerie (20) comprend une source d'imagerie (202) et un imageur (203), un axe central d'un faisceau d'imagerie émis par la source d'imagerie (202) dévie par rapport à un axe de référence, et l'axe de référence est un axe qui passe par le centre d'imagerie (R) et est perpendiculaire à l'imageur (203).

9. Dispositif de radiothérapie selon la revendication 1, dans lequel l'unité d'imagerie (20) comprend un élément de blindage (201) disposé pour être adjacent à l'unité de radiothérapie (10) et configuré pour blinder contre le faisceau de traitement passant par le point d'intersection (I).

10. Dispositif de radiothérapie selon la revendication 9, dans lequel l'élément de blindage (201) est en forme creuse ou en forme de feuille.

11. Dispositif de radiothérapie selon la revendication 1, dans lequel l'unité d'imagerie (20) comprend au moins l'un des suivants :
un appareil à rayons X, un appareil CT, un appareil MRI, un appareil PET, un appareil à ultrasons ou un appareil DSA.

12. Dispositif de radiothérapie selon la revendication 1, comprenant en outre : une porte de blindage (50) configurée pour mettre le dispositif de radiothérapie sous tension ou hors tension, et/ou blinder contre le faisceau de traitement émis par l'unité de radiothérapie (10).

13. Dispositif de radiothérapie selon la revendication 1, dans lequel l'unité de radiothérapie (10) est en forme de bol ou en forme de tube.
